# EUROPEAN PATENT APPLICATION

(11) **EP 1 889 635 A1**
(43) Date of publication of application: **20.02.2008**
(21) Application number: 07472002.0
(22) Date of filing: 06.06.2007
(51) Int. Cl.: A61M 1/28, A61M 1/16, A61K 33/14, A61P 7/08

(54) **Haemodialysis concentrates**

(30) Priority: 08.06.2006 BG 10957406
(71) Applicant: Unipharm AD, 1797 Sofia (BG)
(72) Inventor: Baldev, Tzanko Dimitrov, 1303 Sofia (BG); Daskalov, Angel Dimitrov, 1113 Sofia (BG); Istilyanova, Aneta Yordanova, 1797 Sofia (BG); Chipanov, Dimitar Dimitrov, 1612 Sofia (BG); Mushekov, Valentin Lubomirov, 1000 Sofia (BG)
(74) Representative: Kostadinova, Rossitsa Kirilova

(57) **Abstract**

The present invention relates to haemodialysis concentrates used to make ready-for-use haemodialysis solutions applicable in patients with both acute and chronic renal failure, diabetes and intoxication. The obtained haemodialysis concentrates contain according to the invention electrolytes, a buffer, an osmotic agent, and an antioxidant selected from the group including glutathione, N-acetyl-L-cysteine, and its pharmaceutically acceptable salts or α-lipoic acid and is in dry or liquid form. They are used to prepare a haemodialysis solution to reduce undesirable complications of renal failure including cardiovascular diseases, renal anaemia, renal osteodystrophy, microinflammatory processes, malnutrition, polyneuropathy, and/or encephalopathy. The haemodialysis concentrates and the haemodialysis solutions prepared therefrom according to the present invention are stable and undergo no physicochemical changes and interactions.

## Description

### FIELD OF THE INVENTION

The present invention relates to haemodialysis concentrates used to make ready-for-use haemodialysis solutions applicable in patients with both acute and chronic renal failure, diabetes and intoxication.

### BACKGROUND OF THE INVENTION

Renal failure is a decrease or total loss of renal function which, when chronic, results in a persistent impairment of vital functions of the body and hence in lower survival chances for the patient.

Haemodialysis is a technique used to eliminate the waste products when the kidneys fail to function. The patient's blood is taken from the body and passed through an "artificial kidney" machine and is then returned filtrated to the blood system. The machine includes a dialyser with an inbuilt artificial dialysis membrane that allows for metabolic wastes such as urea, creatinine and other end products of nitrogen metabolism to pass to the dialysis solution by diffusion, osmosis, and filtration. In addition to harmful metabolites, extra water is also removed from the blood though the membrane by vacuum ultrafiltration. This procedure allows for the alkaline acid balance to be recovered and helps remove tissue oedemas and effusions, recover the water electrolyte balance and maintain serum potassium within normal limits.

Cell metabolism involves a continuous generation of reactive oxygen compounds that provoke oxidative stress which is a basic pathogenic mechanism producing adverse effects in the body. During haemodialysis, there is a spontaneously increased production of oxygen radicals which increases oxidative stress significantly. Oxidative stress in itself is responsible for the generation of reactive oxygen, nitrogen and chlorine compounds causing chlorine and nitrogen stress as well.

Due to the nature and specificity of renal failure, several complications occur especially in chronic patients, such as renal anaemia, renal osteodystrophy, symptomatic polyneuropathy, dialysis encephalopathy, malnutrition, microinflammatory processes, cardiovascular diseases including atherosclerosis, ischemia, cardiac hypertrophy, hypotension, hypertension etc. These concomitant diseases are additionally provoked by some disorders associated with renal failure, such as neutropaenia, uraemia and others.

Oxidative stress contributes to the deterioration of the patient's overall condition including general fatigue, reduced workability, decreased psychoemotional tone, frequently persistent itching and impaired life quality. Oxidative stress is a triggering factor for each one of these conditions since its key role in pathogenesis has been proven and it is considered as a major cause of the abovementioned complications of renal failure.

During conventional haemodialysis, the contact with the dialysis membrane generates on the very surface of the membrane an abnormal amount of superoxide radicals, hydroxide and hydrogen peroxide radicals and products of oxidative stress such as malondialdehyde, a low-density oxidised lipoprotein, pathologically oxidised carbohydrate and protein products, complementary fractions and pro-inflammatory cytokines (TNF-alpha, IL-1, IL 6 etc.). They all damage the cell membrane, stimulate protein oxidation and lipid peroxidation, and more generally, speed up physical death.

Several types of dialysis concentrate containing different concentrations of electrolytes, a buffer, and an osmotic agent are currently used to obtain working solutions for conventional haemodialysis. Electrolytes include in particular sodium, potassium, calcium, magnesium, and chorine ions. Bicarbonate and acetate ions are included as buffers and glucose is used as osmotic agent. [Krushkov I., Lambev I. Pharmacotherapeutic Guide, 6^{th} edition and European Pharmacopoeia 5.0; Haemodialysis, solution for, cTp.1700-1703]. Haemodialysis solutions are prepared by conventional concentrate dilution. These conventional concentrates and the haemodialysis solutions obtained therefrom do not help solve the severe problem of oxidative stress.

It is known from US 6627659 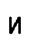 US 6355682 that additional antioxidant is introduced in the body to reduce oxidative stress in renal failure patients on haemodialysis. The substance used as an antioxidant in these cases is N-acetyl -L-cysteine (ACC), directly applied to the patient. In this way, the whole body becomes saturated with the antioxidant. When injected prior to haemodialysis, N-acetyl -L-cysteine as an active substance follows the pharmacokinetics of a "bolus" dose, i.e. with an initial peak of plasma concentration followed by rapid distribution and elimination. A disadvantage of this method is that the antioxidant acts in the blood flow and unevenly over time. When the antioxidant comes in contact with the dialysis membrane, its amount is insufficient to neutralise effectively the increased levels of free radicals in the dialyser which results in a significant increase in the amount of free radicals in the patient's blood. Parallel intravenous infusion during haemodialysis entails antioxidant saturation of the whole body. This allows for relatively balanced plasma concentrations of the active substance to be achieved but at the expense of the central blood circulation. The strength of the antioxidant effect depends on the plasma concentration of the active substance which is the same for the whole distribution, including the contact surface between the blood and the dialysis membrane where oxidation processes occur and develop to the greatest extent during haemodialysis. Again, a disadvantage of this known method is that the antioxidant is administered separately and acts directly in the blood flow which makes it impossible to reduce the amount of free radicals on the very surface of the artificial membrane and thus the amount of free radicals and their products in the patient's blood increases significantly.

### SUMMARY OF THE INVENTION

The problem solved by the present invention is the need to reduce the amount of free radicals resulting from oxidative stress in the blood of patients with renal failure. This is achieved using a haemodialysis concentrate including electrolytes, a buffer, an osmotic agent and an antioxidant. The concentrate is preferably dry or liquid. The antioxidant should be preferably selected from the group including glutathione, N-acetyl -L-cysteine and its pharmaceutically acceptable salts or α-lipoic acid. Preferably, the amount of the antioxidant should range between 0.5 g /l and 4.8 g /l (3.06 mmo/l and 29.41 mmol/l). The concentrate according to the present invention allows for a haemodialysis process in which the amount of free radicals is reduced at the very beginning of dialysis, on the surface of the dialysis membrane. During haemodialysis, the concentrates (diluted to ready-for-use solutions by the dialyser) infiltrate across the dialysis membranes and diffuse therethrough into the patient's body where they exert further their antioxidant effect throughout and after the haemodialysis procedure. The concentrate is stable and undergoes no physicochemical changes and interactions. It can be used to prepare various ready-for-use haemodialysis solutions of various concentrations that comply with state-of-the-art pharmacopoieal requirements. Patients can thus undergo a therapy meeting the trends in modern nephrologic practice. Moreover, the concentrate helps reduce the early effects of oxidative stress and its delayed consequences in renal failure patients such as cardiovascular diseases, renal anaemia, renal osteodystrophy, symptomatic polyneuropathy, dialysis encephalopathy, malnutrition, microinflammatory processes etc.

Electrolytes include for instance water soluble Na, K, Ca, Mg salts, and chlorides in particular. A mixture of sodium acetate and acetic acid or sodium hydrogen carbonate and carbonic acid can be used as a buffer. The osmotic agent can be selected among glucose, polyglucose, glycerol, amino acid, polypeptide, polysorbate or a combination thereof.

The present invention allows for several types of concentrates to be obtained according to the haemodialysis type. In one embodiment, the concentrate is an acid one and is used in bicarbonate haemodialysis. It has the following composition in mmol/l, which is obtained by water dilution in the case of a dry concentrate:

| | | | | |
|---|---|---|---|---|
| Sodium | from | 4550.00 | to | 5075.00 |
| Potassium | from | 0.00 | to | 105.00 |
| Calcium | from | 0.00 | to | 70.00 |
| Magnesium | from | 0.00 | to | 42.00 |
| Chlorine | from | 3150.00 | to | 4200.00 |
| Acetate | from | 87.50 | to | 350.00 |
| Osmotic agent | from | 0.00 | to | 420.00 |
| Antioxidant | from | 3.06 | to | 29.41 |

Another embodiment of the invention is a basic bicarbonate haemodialysis concentrate (8.4%). The concentrate contains sodium hydrogen carbonate in amounts ranging from 595.189 to 1050.000 mmol/l and an antioxidant ranging from 2.50 to 24.00 mmol/l. Both water-diluted dry concentrate and liquid concentrate have a guaranteed content of 1000.0 mmol/l of sodium, 1000.0 mmol/l of hydrogen carbonate and antioxidant within the above limits.

In yet another embodiment, the invention consists in acetate haemodialysis concentrate. It has the following composition in mmol/l, which is obtained by water dilution in the case of a dry concentrate:

| | | | | |
|---|---|---|---|---|
| Sodium | from | 4550.00 | to | 5075.00 |
| Potassium | from | 0.00 | to | 105.00 |
| Calcium | from | 0.00 | to | 70.00 |
| Magnesium | from | 0.00 | to | 42.00 |
| Chlorine | from | 3150.00 | to | 4200.00 |
| Acetate | from | 1120.00 | to | 1575.00 |
| Osmotic agent | from | 0.00 | to | 420.00 |
| Antioxidant | from | 3.06 | to | 29.41 |

The invention also includes a ready-for-use haemodialysis solution obtained by dilution of a concentrate described above. The dilution can be achieved by known methods. This solution may be prepared using various concentrations of ions and an antioxidant depending on the patient's needs. Preferably, the dialysis solution is a diluted mixture of a concentrate according to the invention and a conventional haemodialysis solution. In one embodiment, the solution is a diluted mixture of an acid concentrate according to the present invention and a conventional basic bicarbonate concentrate for bicarbonate haemodialysis. In another embodiment, the solution is a diluted mixture from basic bicarbonate concentrate according to the present invention and a conventional acid concentrate for bicarbonate haemodialysis. In yet another embodiment, the solution is an acetate concentrate according to the invention directly mixed with purified water.

In a preferred embodiment, the haemodialysis solution is aimed at bicarbonate haemodialysis and has the following composition:

| **Ions** | **Haemodialysis solution for bicarbonate haemodialysis mmol/l** | | | |
|---|---|---|---|---|
| Sodium | from | 130.00 | to | 145.00 |
| Potassium | from | 0.00 | to | 3.00 |
| Calcium | from | 0.00 | to | 2.00 |
| Magnesium | from | 0.00 | to | 1.20 |
| Acetate | from | 2.50 | to | 10.00 |
| Chlorides | from | 90.00 | to | 120.00 |
| Osmotic agent | from | 0.00 | to | 12.00 |
| Hydrocarbonates | from | 19.18 | to | 36.75 |
| Antioxidant | from | 0.088 | to | 0.840 |

In another embodiment of the invention, the solution for acetate haemodialysis has the following composition:

| **Ions** | **Haemodialysis solution for acetate haemodialysis mmol/l** | | | |
|---|---|---|---|---|
| Sodium | from | 130.000 | to | 145.000 |
| Potassium | from | 0.000 | to | 3.000 |
| Calcium | from | 0.000 | to | 2.000 |
| Magnesium | from | 0.000 | to | 1.200 |
| Acetate | from | 32.000 | to | 45.000 |
| Chlorides | from | 90.000 | to | 120.000 |
| Glucose | from | 0.000 | to | 12.000 |
| Antioxidant | from | 0.088 | to | 0.840 |

The concentrates according to the invention are used to prepare haemodialysis solutions aimed at reducing undesirable complications of renal failure. Targeted complications include cardiovascular diseases, renal anaemia, renal osteodystrophy, microinflammatory processes, malnutrition, polyneuropathy, and/or encephalopathy in patients on haemodialysis.

The haemodialysis concentrates and haemodialysis solution prepared therefrom according to the invention are stable and undergo no physicochemical changes and interactions. They are meant for use in haemodialysis to reduce oxidative stress in both adults and children with acute or chronic renal failure, diabetes, intoxications, and concomitant complications.

The haemodialysis solutions according to the present invention interact with the patient's blood and respectively with the patient's body and eliminate wastes from the blood through the dialyser membranes by diffusion, osmosis, and filtration. They infiltrate across the dialysis membranes so that at every moment of the haemodialysis procedure the concentration of fresh working solution, including that of the antioxidant on the blood surface of the membrane, is maintained at its highest and constant at the same time. This make it possible to achieve a "steady state" throughout the haemodialysis procedure by effective neutralization of free radicals on the dialysis membrane where their formation is most intensive, thus reducing significantly oxidative stress and its delayed consequences.

In addition to their infiltration across the dialysis membrane during haemodialysis, solutions diffuse therethrough and penetrate into the body to exert further their antioxidant effect.

The haemodialysis concentrates and the haemodialysis solutions prepared therefrom according to the present invention reduce cardiovascular risk by decreasing oxidative stress and its delayed consequences, more specifically by slowing down the evolution of atherosclerosis, including by reducing the formation of harmful oxidized fractions of cholesterol, carbohydrates, and proteins. Additional effects in renal failure patients on haemodialysis include improved anaemic syndrome, reduced need for treatment with erythropoietic stimulating agents, postponed renal osteodystrophy, improved general clinical status, and reduced morbidity and lethality ratios. The administration of the concentrates and solutions according to the invention reduces the direct and indirect costs associated with the treatment of renal failure patients on haemodialysis since there is no need for special preparatory measures and manipulations other than those conventionally required for a haemodialysis procedure.

At the same time, the antioxidant-containing haemodialysis solutions according to the present invention make it possible to avoid the intravenous application of a large amount of antioxidant that entails a risk of undesirable side effects. They also help eliminate the need for a prolonged antioxidant infusion during haemodialysis which is otherwise necessary to maintain effective plasma concentrations since antioxidants metabolise rapidly. Last but not least, the use of the more expensive ampoule form for intravenous antioxidant application is also avoided.

### EXAMPLES

The examples of antioxidant-containing haemodialysis concentrates (HDC) according to the invention are given as dry or liquid. The examples and embodiments described below are only an illustration and do not set limits to the invention concept, its coverage being determined by that of the patent claims. The amounts of therapeutic substance and the rest of the ingredients are given in the following tables:

### EXAMPLE Nº 1A.

Acid concentrate for bicarbonate haemodialysis:

| | **Dry HDC** | **Liquid HDC** |
|---|---|---|
| **Raw material** | **Amount, kg** | **Amount, kg** |
| Sodium chloride Eur Ph 2005 | 214.800 | 214.800 |
| Potassium chloride Eur Ph 2005 | 7,830 | 7.830 |
| Calcium chloride .6H₂O Eur Ph 2005 | 13.410 | 13.410 |
| Magnesium chloride .6H₂O Eur Ph 2005 | 3.560 | 3.560 |
| Ice-acetic acid | 4.200 | 4.200 |
| ACC | 2.040 | 2.040 |
| Purified water - Eur Ph 2005 | - | to 1000.0 l |

### EXAMPLE Nº 1 B.

Acid concentrate for bicarbonate haemodialysis:

| | **Dry HDC** | **Liquid HDC** |
|---|---|---|
| **Raw material** | **Amount, kg** | **Amount, kg** |
| Sodium chloride Eur Ph 2005 | 214.800 | 214.800 |
| Potassium chloride Eur Ph 2005 | 7,830 | 7.830 |
| Calcium chloride .6H₂O Eur Ph 2005 | 13.410 | 13.410 |
| Magnesium chloride .6H₂O Eur Ph 2005 | 3.560 | 3.560 |
| Ice-acetic acid | 4.200 | 4.200 |
| Glutathione | 2.040 | 2.040 |
| Purified water - Eur Ph 2005 | - | to 1000.0 l |

### EXAMPLE Nº 2A.

Acid concentrate for bicarbonate haemodialysis:

| | **Dry HDC** | **Liquid HDC** |
|---|---|---|
| **Raw material** | **Amount, kg** | **Amount, kg** |
| Sodium chloride Eur Ph 2005 | 214.800 | 214.800 |
| Potassium chloride Eur Ph 2005 | 7.830 | 7.830 |
| Calcium chloride .6H₂O Eur Ph 2005 | 13.410 | 13.410 |
| Magnesium chloride .6H₂O Eur Ph 2005 | 3.560 | 3.560 |
| Ice-acetic acid | 4.200 | 4,200 |
| Glucose monohydrate | 42.000 | 42.000 |
| ACC Eur Ph 2005 | 2.040 | 2.040 |
| Purified water Eur Ph 2005 | - | to 1000.00 l |

### EXAMPLE Nº 2B.

Acid concentrate for bicarbonate haemodialysis:

| | **Dry HDC** | **Liquid HDC** |
|---|---|---|
| **Raw material** | **Amount, kg** | **Amount, kg** |
| Sodium chloride Eur Ph 2005 | 214.800 | 214.800 |
| Potassium chloride Eur Ph 2005 | 7.830 | 7.830 |
| Calcium chloride .6H₂O Eur Ph 2005 | 13.410 | 13.410 |
| Magnesium chloride .6H₂O Eur Ph 2005 | 3.560 | 3.560 |
| Ice-acetic acid | 4.200 | 4,200 |
| Polysorbate | 42.000 | 42.000 |
| Glutathione Eur Ph 2005 | 2.040 | 2.040 |
| Purified water Eur Ph 2005 | - | to 1000.00 l |

### EXAMPLE Nº3A

Bicarbonate concentrate for haemodialysis (8.4%):

| | **Dry HDC** | **Liquid HDC** |
|---|---|---|
| **Raw material** | **Amount, kg** | **Amount, kg** |
| NaHCO₃ Eur Ph 2005 | 84.000 | 84.000 |
| ACC Eur Ph 2005 | 2.500 | 2.500 |
| Purified water Eur Ph 2005 | - | to 1000.00 l |

### EXAMPLE Nº3B

Bicarbonate concentrate for haemodialysis (8.4%):

| | **Dry HDC** | **Liquid HDC** |
|---|---|---|
| **Raw material** | **Amount, kg** | **Amount, kg** |
| NaHCO₃ Eur Ph 2005 | 84.000 | 84.000 |
| Glutathione Eur Ph 2005 | 2.500 | 2.500 |
| Purified water Eur Ph 2005 | - | to 1000.00 l |

### EXAMPLE Nº4A

Concentrate for acetate haemodialysis:

| | **Dry HDC** | **Liquid HDC** |
|---|---|---|
| **Raw material** | **Amount, kg** | **Amount, kg** |
| Sodium chloride Eur Ph 2005 | 204.600 | 204.600 |
| Potassium chloride Eur Ph 2005 | 7.800 | 7.800 |
| Calcium chloride .6H₂O Eur Ph 2005 | 13.400 | 13.400 |
| Magnesium chloride .6H₂O Eur Ph 2005 | | |
| Sodium acetate .3H₂O Eur Ph 2005 | 181.000 | 181.000 |
| ACC Eur Ph 2005 | 2.400 | 2.400 |
| Purified water - Eur Ph 2005 | - | to 1000.00 l |

### EXAMPLE Nº4B

Concentrate for acetate haemodialysis:

| | **Dry HDC** | **Liquid HDC** |
|---|---|---|
| **Raw material** | **Amount, kg** | **Amount, kg** |
| Sodium chloride Eur Ph 2005 | 204.600 | 204.600 |
| Potassium chloride Eur Ph 2005 | 7.800 | 7.800 |
| Calcium chloride .6H₂O Eur Ph 2005 | 13.400 | 13.400 |
| Magnesium chloride .6H₂O Eur Ph 2005 | | |
| Sodium acetate .3H₂O Eur Ph 2005 | 181.000 | 181.000 |
| Glutathione Eur Ph 2005 | 2.400 | 2.400 |
| Purified water - Eur Ph 2005 | - | to 1000.00 l |

### EXAMPLE Nº5A

Concentrate for acetate haemodialysis:

| | **Dry HDC** | **Liquid HDC** |
|---|---|---|
| **Raw material** | **Amount, kg** | **Amount, kg** |
| Sodium chloride Eur Ph 2005 | 204.600 | 204.600 |
| Potassium chloride Eur Ph 2005 | 7.800 | 7.800 |
| Calcium chloride .6H₂O Eur Ph 2005 | 13.400 | 13.400 |
| Magnesium chloride .6H₂O Eur Ph 2005 | | |
| Sodium acetate .3H₂O Eur Ph 2005 | 181.000 | 181.000 |
| Polysorbate Eur Ph 2005 | 42.000 | 42.000 |
| ACC Eur Ph 2005 | 2.400 | 2.400 |
| Purified water - Eur Ph 2005 | - | to 1000.00 l |

### EXAMPLE Nº5B

Concentrate for acetate haemodialysis:

| | **Dry HDC** | **Liquid HDC** |
|---|---|---|
| **Raw material** | **Amount, kg** | **Amount, kg** |
| Sodium chloride Eur Ph 2005 | 204.600 | 204.600 |
| Potassium chloride Eur Ph 2005 | 7.800 | 7.800 |
| Calcium chloride .6H₂O Eur Ph 2005 | 13.400 | 13.400 |
| Magnesium chloride .6H₂O Eur Ph 2005 | | |
| Sodium acetate .3H₂O Eur Ph 2005 | 181.000 | 181.000 |
| Glucose monohydrate Eur Ph 2005 | 42.000 | 42.000 |
| Glutathione Eur Ph 2005 | 2.400 | 2.400 |
| Purified water - Eur Ph 2005 | - | to 1000.00 l |

**EXAMPLES NºNº 6, 7** 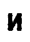 **8**

Haemodialysis solutions for bicarbonate haemodialysis prepared from concentrates for haemodialysis according to Examples 1 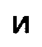 2 by mixing with conventional antioxidant-free hydrogen carbonate solution (mmol/l):

| | **Example 6A** | **Example 7A** | **Example 8A** |
|---|---|---|---|
| Sodium | 130.00 | 130.00 | 145.00 |
| Potassium | 0.00 | 1,50 | 3.00 |
| Calcium | 0.00 | 1.00 | 2.00 |
| Magnesium | 0.00 | 0.60 | 1.20 |
| Acetic acid | 2.50 | 6.25 | 10.00 |
| Chlorides | 90.00 | 105,00 | 120.00 |
| Glucose | 0.00 | 6.00 | 12.00 |
| Hydrogen carbonates | 19.18 | 27.97 | 36.75 |
| A C C | 0.09 | 0.42 | 0.84 |
| | | | |

| | **Example 6b** | **Example 7B** | **Example 8B** |
|---|---|---|---|
| Sodium | 130.00 | 130.00 | 145.00 |
| Potassium | 0.00 | 1,50 | 3.00 |
| Calcium | 0.00 | 1.00 | 2.00 |
| Magnesium | 0.00 | 0.60 | 1.20 |
| Acetic acid | 2.50 | 6.25 | 10.00 |
| Chlorides | 90.00 | 105,00 | 120.00 |
| Polysorbate | 0.00 | 6.00 | 12.00 |
| Hydrogen carbonates | 19.18 | 27.97 | 36.75 |
| Glutathione | 0.09 | 0.42 | 0.84 |

**EXAMPLES NºNº 9, 10** 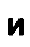 **11**

Haemodialysis solutions for bicarbonate haemodialysis prepared from conventional antioxidant-free concentrate for haemodialysis by mixing with antioxidant-containing hydrogen carbonate solution according to Example 3 (mmol/l):

| | **Example 9A** | **Example 10A** | **Example 11 A** |
|---|---|---|---|
| Sodium | 130.00 | 130.00 | 145.00 |
| Potassium | 0.00 | 1,50 | 3.00 |
| Calcium | 0.00 | 1.00 | 2.00 |
| Magnesium | 0.00 | 0.60 | 1.20 |
| Acetic acid | 2.50 | 6.25 | 10.00 |
| Chlorides | 90.00 | 105,00 | 120.00 |
| Glucose | 0.00 | 6.00 | 12.00 |
| Hydrogen carbonates | 19.18 | 27.97 | 36.75 |
| A C C | 0.09 | 0.42 | 0.84 |
| | | | |

| | **Example 9B** | **Example 10B** | **Example 11 B** |
|---|---|---|---|
| Sodium | 130.00 | 130.00 | 145.00 |
| Potassium | 0.00 | 1,50 | 3.00 |
| Calcium | 0.00 | 1.00 | 2.00 |
| Magnesium | 0.00 | 0.60 | 1.20 |
| Acetic acid | 2.50 | 6.25 | 10.00 |
| Chlorides | 90.00 | 105,00 | 120.00 |
| Polysorbate | 0.00 | 6.00 | 12.00 |
| Hydrogen carbonates | 19.18 | 27.97 | 36.75 |
| Glutathione | 0.09 | 0.42 | 0.84 |

**EXAMPLES NºNº 12, 13** 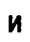 **14**

Haemodialysis solutions for acetate haemodialysis prepared from haemodialysis concentrates according to Examples 4 and 5 (mmol/l)

| | **Example 12A** | **Example 13A** | **Example 14A** |
|---|---|---|---|
| Sodium | 130.00 | 138.00 | 145.00 |
| Potassium | 0.00 | 1,50 | 3.00 |
| Calcium | 0.00 | 1.00 | 2.00 |
| Magnesium | 0.00 | 0.60 | 1.20 |
| Acetates | 32.50 | 38,50 | 45.00 |
| Chlorides | 90.00 | 105,00 | 120.00 |
| Polysorbate | 0.00 | 6.00 | 12.00 |
| A C C | 0.09 | 0.42 | 0.84 |
| | | | |

| | **Example 12B** | **Example 13B** | **Example 14B** |
|---|---|---|---|
| Sodium | 130.00 | 138.00 | 145.00 |
| Potassium | 0.00 | 1,50 | 3.00 |
| Calcium | 0.00 | 1.00 | 2.00 |
| Magnesium | 0.00 | 0.60 | 1.20 |
| Acetates | 32.50 | 38,50 | 45.00 |
| Chlorides | 90.00 | 105,00 | 120.00 |
| Glucose | 0.00 | 6.00 | 12.00 |
| Glutathione | 0.09 | 0.42 | 0.84 |

### EXAMPLE Nº15

### Antioxidant effect of created concentrates for haemodialysis containing N-acetyl-L-cysteine (from 0.5 to 4.8 g/l)

Oxidants are highly reactive compounds with only a few seconds of half-life. Therefore, their detection in vivo is impossible. They are detected using other markers such as malondialdehyde (MDA) as a basic reaction product of lipid peroxidation.

A trial was conducted with ten chronic renal failure patients on scheduled haemodialysis treatment. They were treated by haemodialysis (HD-1) using conventional haemodialysis concentrate. Blood samples from the patients were collected prior to haemodialysis (hour 0), at the end of the first hour (hour 1) and at the end of the forth hour (hour 4), i.e. at the end of the HD session to evaluate malondialdehyde levels as a quantitative marker of oxidative stress. The next haemodialysis session HD-2 was performed two days later with the same patients. The haemodialysis procedures were carried out using a HDC containing 2.4 g/l of N-acetyl L-cysteine. Again, blood samples from the patients undergoing HD-2 were analysed for MDA according to the above scheme. The following averaged results were obtained:
**MDA umol/l in HD-1: hour0-0.644 hour1-0.831 hour4-0.727;** An increase of 0.187 (29%) in MDA levels was detected at hour1 compared to hour0. The increase at hour4 compared to hour0 was 0.08 (12.8%).
**MDA umol/l in HD -2 with ACC: hour 0-0.812 hour 1-0.632 hour 4-0.545;** A decrease of 0.173 (21.3%) in MDA levels was detected at hour1 compared to hour0. The decrease at hour4 compared to hour0 was 0.267 (32.8%).

### Conclusions:

During haemodialysis using a conventional concentrate the levels of malondialdehyde increased significantly which indicated the development of oxidative stress. During haemodialysis using an antioxidant-containing concentrate according to the invention, malondialdehyde levels decreased significantly, i.e. the development of oxidative stress was reduced. Oxidative stress reduction was found to be more significant in patients with lower body weight than with those with higher body weight. In patients with higher body weight it is appropriate to use a haemodialysis concentrate containing a greater amount of antioxidant per litre.

### EXAMPLE Nº 16

**Table Nº1**

| **Analysing the stability of a basic bicarbonate concentrate for haemodialysis, containing N-acetyl-L-cysteine (ACC) as antioxidant, obtained according to Example 3A** | | | | | | |
|---|---|---|---|---|---|---|
| 8.4% Bicarbonate | | | | | | |
| **Lot Nº C8-031204** | | | | | | |
| Primary package: A 5 / 6-liter bottle / PET/ | | | | | | |
| Storage conditions: 20-25°C / 60 % | | | | | | |
| Lab samples | | | | | | |
| | Appearance | pH | Composition mmol/I | | | Microbiological analysis / Bacterial endotoxins |
| | | | Na | HCO₃ | ACC | |
| Initial analysis | conforms | 7.98 | 1005.23 | 1004.80 | 12.87 | conforms / conforms |
| 3 months | conforms | 8.17 | 1005.23 | 1004.80 | 12.75 | conforms / conforms |
| 6 months | conforms | 8.24 | 1005.23 | 1004.80 | 12.38 | conforms / conforms |
| 9 months | conforms | 8.36 | 1018.71 | 1018.08 | 11.83 | conforms / conforms |
| 12 months | conforms | 8.49 | 1025.00 | 1025.61 | 11.64 | conforms / conforms |
| 18 months | conforms | 8.54 | 1025.00 | 1025.61 | 11.58 | conforms / conforms |

### EXAMPLE Nº17

**Table Nº2**

| **Analysing the stability of an acid bicarbonate concentrate for haemodialysis, containing N-acetyl-L-cysteine (ACC) as an antioxidant, obtained according to Example 1A** | | | | | | | | | | |
|---|---|---|---|---|---|---|---|---|---|---|
| Acid A 13. (1) | | | | | | | | | | |
| **Lot Nº C7-021104** | | | | | | | | | | |
| Primary package: A 5-liter bottle / PET/ | | | | | | | | | | |
| Storage conditions: 20-25°C / 60 % | | | | | | | | | | |
| Lab samples | | | | | | | | | | |
| | Appearance | pH | Composition mmol/I | | | | | | | Microbiological analysis / Bacterial endotoxins |
| | | | Na | K | Ca | Mg | Cl | Acetate | ACC | |
| Initial analysis | conforms | 1.82 | 3665.10 | 105.63 | 61.13 | 17.69 | 3909.97 | 70.29 | 15.32 | conforms/conforms |
| 3 months | conforms | 1.90 | 3668.15 | 104.86 | 61.38 | 16.87 | 3920.97 | 70.46 | 15.01 | conforms/conforms |
| 6 months | conforms | 1.96 | 3675.98 | 104.86 | 61.38 | 17.28 | 3928.30 | 70.79 | 14.77 | conforms/conforms |
| 9 months | conforms | 1.88 | 3668.58 | 105.38 | 61.38 | 17.28 | 3935.92 | 70.29 | 14.58 | conforms/conforms |
| 12 months | conforms | 1.92 | 3702.51 | 106.14 | 61.63 | 17.69 | 3955.10 | 70.29 | 14.64 | conforms/conforms |
| 18 months | conforms | 1.86 | 3701.21 | 106.40 | 61.88 | 17.69 | 3959.89 | 70.62 | 14.52 | conforms/conforms |
| 24 months | conforms | 1.91 | 3716.43 | 106.65 | 62.13 | 17.69 | 3973.15 | 70.79 | 14.46 | conforms/conforms |

### EXAMPLE Nº 18

**Table Nº3**

| **Analysing the stability of an acid concentrate for bicarbonate haemodialysis, containing glucose and N-acetyl-L-cysteine (ACC) as antioxidant, obtained according to Example 2A** | | | | | | | | | | | |
|---|---|---|---|---|---|---|---|---|---|---|---|
| HDC-A14 | | | | | | | | | | | |
| **Lot Nº C9-011004** | | | | | | | | | | | |
| Primary package: A 5-liter bottle / PET/ | | | | | | | | | | | |
| Storage conditions: 20-25°C / 60 % | | | | | | | | | | | |
| Lab samples | | | | | | | | | | | |
| | Appearance | pH | Composition mmol/I | | | | | | | | Microbiological analysis / Bacterial endotoxins |
| | | | Na | K | Ca | Mg | Cl | Acetate | Glucose | ACC | |
| Initial analysis | conforms | 1.76 | 3665.54 | 103.84 | 60.63 | 25.92 | 3961.30 | 69.95 | 209.01 | 15.63 | conforms/conforms |
| 3 months | conforms | 1.81 | 3668.15 | 104.86 | 61.38 | 25.92 | 3964.69 | 70.62 | 208.66 | 14.89 | conforms/conforms |
| 6 months | conforms | 1.93 | 3687.72 | 102.82 | 61.88 | 26.74 | 3976.53 | 69.78 | 206.49 | 14.64 | conforms/conforms |
| 9 months | conforms | 1.89 | 3678.15 | 107.17 | 61.88 | 27.15 | 3983.58 | 70.79 | 202.50 | 14.71 | conforms/conforms |
| 12 months | conforms | 1.84 | 3695.55 | 108.96 | 62.62 | 27.15 | 3996.28 | 71.47 | 201.44 | 14.58 | conforms/conforms |
| 18 months | conforms | 1.92 | 3719.48 | 109.72 | 62.38 | 27.57 | 4010.10 | 70.96 | 199.77 | 14.40 | conforms/conforms |

### EXAMPLE Nº 19

**Table Nº4**

| **Analysing the stability of a concentrate for acetate haemodialysis, containing N-acetyl-L-cysteine (ACC) as an antioxidant, obtained according to Example 4A** | | | | | | | | | | |
|---|---|---|---|---|---|---|---|---|---|---|
| HDC-3 | | | | | | | | | | |
| **Lot Nº C3-040204** | | | | | | | | | | |
| Primary package: A 5-liter bottle / PET/ | | | | | | | | | | |
| Storage conditions: 20-25°C / 60 % | | | | | | | | | | |
| Lab samples | | | | | | | | | | |
| | Appearance | pH | Composition mmol/l: | | | | | | | Microbiological analysis / Bacterial endotoxins |
| | | | Na | K | Ca | Mg | Cl | Acetate | ACC | |
| Initial analysis | conforms | 6.34 | 4880.43 | 104.61 | 62.62 | 25.92 | 3790.09 | 1337.97 | 15.32 | conforms/conforms |
| 3 months | conforms | 6.35 | 4880.17 | 104.61 | 62.62 | 25.92 | 3807.86 | 1354.91 | 15.18 | conforms/conforms |
| 6 months | conforms | 6.33 | 4889.12 | 105.12 | 62.87 | 25.92 | 3811.52 | 1354.06 | 14.96 | conforms/conforms |
| 9 months | conforms | 6.18 | 4896.06 | 106.14 | 63.37 | 26.33 | 3812.65 | 1358.13 | 14.88 | conforms/conforms |
| 12 months | conforms | 6.04 | 4916.96 | 106.91 | 63.37 | 26.33 | 3827.88 | 1360.67 | 14.57 | conforms/conforms |
| 18 months | conforms | 5.97 | 4916.96 | 106.65 | 63.62 | 26.33 | 3833.24 | 1368.97 | 14.51 | conforms/conforms |
| 24 months | conforms | 5.90 | 4937.84 | 107.17 | 63.62 | 26.74 | 3835.22 | 1369.94 | 14.40 | conforms/conforms |

## Claims

1. A haemodialysis concentrate containing electrolytes, a buffer, and an osmotic agent, **characterised by** the fact that it contains an antioxidant.

2. A concentrate according to Claim 1, **characterized by** the fact that the antioxidant is selected from the group including glutathione, N-acetyl-L-cysteine and its pharmaceutically acceptable salts or α-lipoic acid.

3. A concentrate according to Claim 1 **characterised by** the fact that the amount of antioxidant ranges from 0.5 g/l to 4.8 g/l (from 3.06 mmo/l to 29.41 mmol/l).

4. A concentrate according to any of the above claims, **characterised by** being dry or liquid.

5. A concentrate according to Claim 4, **characterised by** being liquid and comprising the following pharmaceutical ingredients (mmol/l):
| | | | | |
|---|---|---|---|---|
| Sodium | from | 4550.00 | to | 5075.00 |
| Potassium | from | 0.00 | to | 105.00 |
| Calcium | from | 0.00 | to | 70.00 |
| Magnesium | from | 0.00 | to | 42.00 |
| Chlorine | from | 3150.00 | to | 4200.00 |
| Acetate | from | 87.50 | to | 350.00 |
| Osmotic agent | from | 0.00 | to | 420.00 |
| Antioxidant | from | 3.06 | to | 29.41 |

6. A concentrate according to Claim 5, **characterised by** the fact that the pharmaceutical composition is obtained by dilution of dry concentrate in water.

7. A concentrate according to Claim 4, **characterized by** being liquid and by containing sodium hydrogen carbonate ranging from 595.189 mmol/l to 1050.00 mmol/l and an antioxidant ranging from 2.50 mmol/l to 24.00 mmol/l.

8. A concentrate according to Claim 7, **characterised by** being obtained by dilution of dry concentrate in water.

9. A concentrate according to Claim 4, **characterised by** being liquid and containing the following pharmaceutical ingredients (mmol/l):
| | | | | |
|---|---|---|---|---|
| Sodium | from | 4550.00 | to | 5075.00 |
| Potassium | from | 0.00 | to | 105.00 |
| Calcium | from | 0.00 | to | 70.00 |
| Magnesium | from | 0.00 | to | 42.00 |
| Chlorine | from | 3150.00 | to | 4200.00 |
| Acetate | from | 1120.00 | to | 1575.00 |
| Osmotic agent | from | 0.00 | to | 420.00 |
| Antioxidant | from | 3.06 | to | 29.41 |

10. A concentrate according to Claim 9, **characterised by** the fact that the pharmaceutical composition is obtained by dilution of dry concentrate in water.

11. A solution for haemodialysis containing a diluted conventional concentrate, **characterised by** containing also a diluted concentrate according to any of the above claims.

12. A solution according to Claim 11, **characterized by** including a mixture of a diluted concentrate according to Claim 5 or Claim 6 and a conventional basic bicarbonate concentrate.

13. A solution according to Claim 12, **characterised by** the following composition (mmol/l):
| | | | |
|---|---|---|---|
| Sodium | 130.000 | to | 145.000 |
| Potassium | 0.000 | to | 3.000 |
| Calcium | 0.000 | to | 2.000 |
| Magnesium | 0.000 | to | 1.200 |
| Chlorine | 90.000 | to | 120.000 |
| Osmotic agent | 0.000 | to | 12.000 |
| Acetate | 2.500 | to | 10.000 |
| Hydrogen carbonate | 19.180 | to | 36.750 |
| Antioxidant | 0.088 | to | 0.840 |

14. A solution according to Claim 11, **characterized by** including a mixture of a diluted concentrate according to Claim 7 or 8 and a conventional acid bicarbonate concentrate.

15. A solution according to Claim 14, **characterized by** the following composition (mmol/l):
| | | | |
|---|---|---|---|
| Sodium | 130.000 | to | 145.000 |
| Potassium | 0.000 | to | 3.000 |
| Calcium | 0.000 | to | 2.000 |
| Magnesium | 0.000 | to | 1.200 |
| Chlorine | 90.000 | to | 120.000 |
| Osmotic agent | 0.000 | to | 12.000 |
| Acetate | 2.500 | to | 10.000 |
| Hydrogen carbonate | 19.180 | to | 36.750 |
| Antioxidant | 0.088 | to | 0.840 |

16. A solution for haemodialysis **characterized by** containing a diluted concentrate according to claim 9 or 10.

17. A solution according to Claim16, **characterized by** the following composition (mmol/l):
| | | | | |
|---|---|---|---|---|
| Sodium | from | 130.000 | to | 145.000 |
| Potassium | from | 0.000 | to | 3.000 |
| Calcium | from | 0.000 | to | 2.000 |
| Magnesium | from | 0.000 | to | 1.200 |
| Acetates | from | 32.000 | to | 45.000 |
| Chlorides | from | 90.000 | to | 120.000 |
| Glucose | from | 0.000 | to | 12.000 |
| Antioxidant | from | 0.088 | to | 0.840 |

18. Use of the concentrate according to any of Claims 1 through 10 to prepare a haemodialysis solution to reduce the undesirable complications of renal failure.

19. Use according to Claim 18, where undesirable complications of renal failure include cardiovascular diseases, renal anaemia, renal osteodystrophy, microinflammatory processes, malnutrition, polyneuropathy, and/or encephalopathy in patients on haemodialysis.
